# EUROPEAN PATENT APPLICATION

(11) **EP 0 722 701 A1**
(43) Date of publication of application: **24.07.1996**
(21) Application number: 96200111.1
(22) Date of filing: 18.01.1996
(51) Int. Cl.: A61F 2/06

(54) **Y-shaped stent and method for positioning such a stent**

(30) Priority: 19.01.1995 NL 9500094
(71) Applicant: CORDIS EUROPA N.V., NL-9301 LJ Roden (NL)
(72) Inventor: Glastra, Hendrik, NL-7535 PG Enschede (NL)
(74) Representative: 't Jong, Bastiaan Jacobus

(57) **Abstract**

The invention provides a Y-shaped stent, in particular one which is used to reinforce the bifurcation of the aorta into the arteries leading to the lower limbs respectively, to replace a bifurcation prosthesis implanted during conventional surgery.
This Y-shaped stent comprises a first stent arranged inside the aorta at the level of the bifurcation and at least two cylindrical stents, each of which is placed with a first section inside an artery branching off and with the remaining section inside the lumen of the first stent.

## Description

The invention relates to a Y-shaped stent, in particular one which is used to reinforce the bifurcation of the aorta into the arteries leading to the lower limbs respectively, and a method for positioning such a stent.

Such a stent can be employed in an advantageous manner to reinforce the bifurcation of the aorta into the two smaller arteries leading to the lower limbs. With the present state of technology such a stent is not available and, in the case of a lesion, it is necessary to replace the entire section of the blood vessel by a new, artificial blood vessel section (the bifurcation prosthesis). The surgery involved, lasting several hours, entails a high degree of risk and is a great burden to the patient.

The stent according to the invention, which makes this operation superfluous, is characterised by a first stent placed inside the aorta at the level of the bifurcation and at least two cylindrical stents, each of which is placed with a first section in an artery branching off, and with the remaining section inside the lumen of the first stent.

Preferred embodiments of this stent are characterised in claims 2-4 inclusive.

Of the Y-shaped stent according to the invention, the first stent is fixed securely in the artery which divides into two branches; the cylindrical stents positioned both inside this first stent and inside the arteries branching off, are fixed in a reliable and sealing manner in the first stent on the one side and in the artery concerned on the other.

In particular when stents are used which are known from EP-A-0 521 573 and EP-A-0 617 930, both in the name of the Applicant, a reliable and sealed anchoring is obtained as a result of the specific configuration of this stent known as such, which adapts properly and in a sealing manner to the wall against which it is positioned.

The exclusive right applied for also encompasses a method for forming such a stent as characterised in claims 5-7 inclusive.

The invention will be explained in greater detail with reference to the attached drawings.

Figure 1 illustrates schematically the aorta and the arteries branching off to the lower limbs.

Figure 2 shows how, according to the invention, a first stent has been placed inside the aorta.

Figure 3 shows how, using the techniques and stent known from EP-A-0 521 573 and EP-A-0 617 930, a cylindrical stent, still in the unexpanded state, has been arranged inside the first stent and each of the two arteries branching off.

Figure 4 shows the complete Y-shaped stent following expansion and curing of the two cylindrical stents.

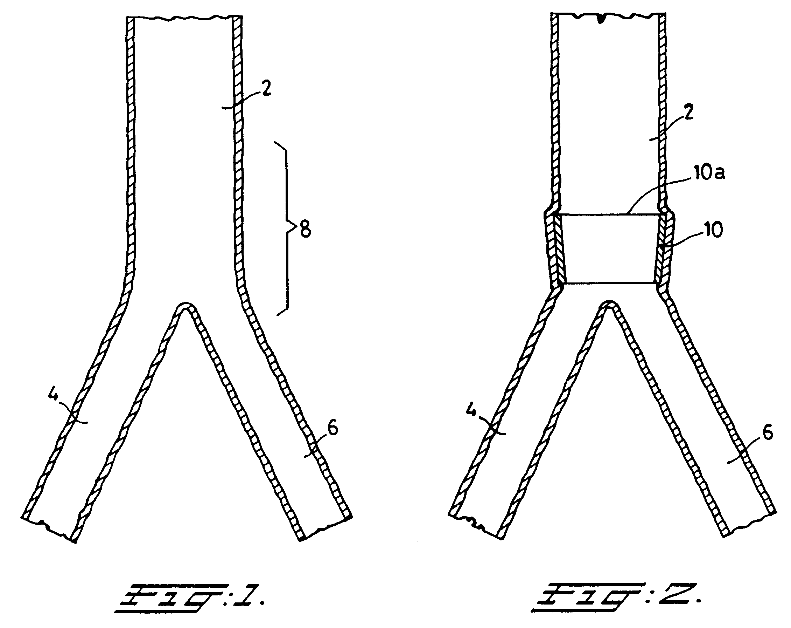

Figure 1 illustrates schematically and in a longitudinal cross-section the aorta 2 and the two smaller arteries 4, 6 branching off and leading to the lower limbs respectively. It is assumed that the region of the bifurcation, in other words the area 4 indicated by the bracket 8, is affected, in which case either the wall of the vessel is impaired locally or the lumen of the vessels is occluded to a greater or lesser degree. So far such a lesion could only be cured by replacing both the bifurcation and the sections of the arteries with artificial blood vessels (the bifurcation prosthesis), involving major surgery and a high degree of risk.

With the stent and the method according to the invention respectively, such an operation has become superfluous.

According to the invention first of all a first stent 10 is placed inside the aorta 2. The Figures show a stent 10 with a cone-shaped lumen (which is preferred, although also another, suitably shaped lumen can be employed). The stent 10 is of the type described in EP-A-0 521 573 and EP-A-0 617 930 and is positioned by means of the method described in these publications, whereby the lumen 20a with the largest diameter is directed away from the arteries 4 and 6 branching off.

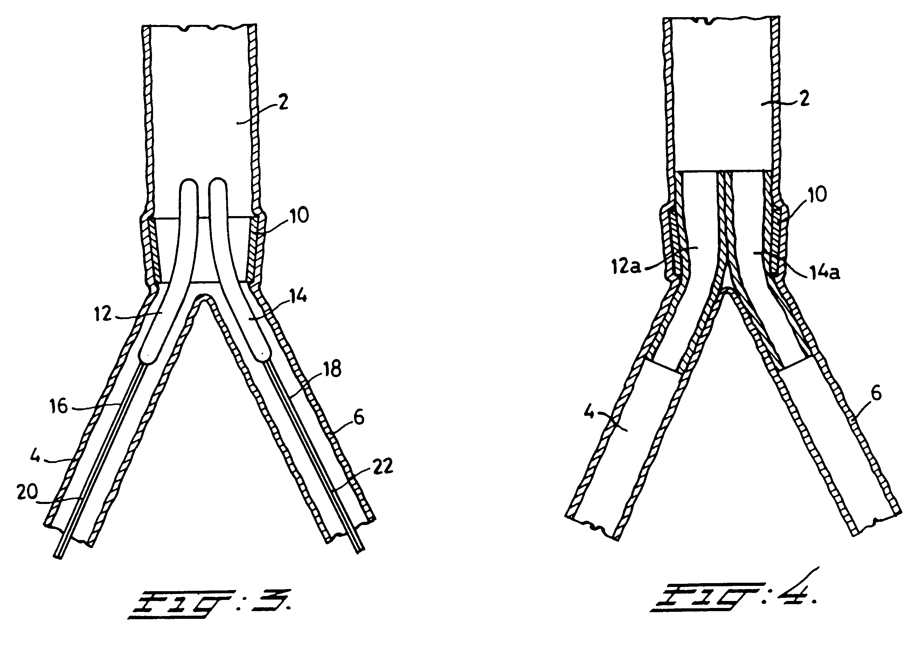

Next a semimanufacture of a cylindrical stent, known from the publications referred to above and indicated in Figure 3 with the numbers 12 and 14 respectively, is placed, via the arteries 4, 6 branching off respectively, inside the sections of these arteries close to the bifurcation and inside the stent 10. Each semimanufacture is made up of an expandable carrier balloon connected to an accessory catheter 16, 18 respectively and, arranged around it, a sleeve-shaped cylindrical stent which expands on expansion of the carrier balloon. By irradiating the curable material inside the expandable sleeve by means of suitable radiation (e.g. UV radiation), supplied via the optic fibres 20, 22 respectively, the material will cure and the ultimately complete stent will be formed which will remain firmly anchored in position after withdrawal of the deflated carrier balloon. The stent concerned and the method employed have been described in detail in the publications referred to above and are considered to form part of this.

Finally, the complete Y-shaped stent and the situation as illustrated in Figure 4 are obtained.

The stent 10 fixed inside the aorta 2 receives the upper sections of the two cylindrical stents 12a, 14a of which the lower sections are fixed inside the arteries 4, 6 branching off respectively. As a result of the specific construction of the expandable stents employed which, in expanded state, adapt perfectly to the shape of the blood vessels and lumen of the first stent 10 respectively, it is ensured that both stents make a close, sealing contact with the inner wall of the aorta 2, the inner wall of the stent 10 and the inner walls of the arteries 4, 6 respectively.

## Claims

1. Y-shaped stent, particularly used for the purpose of reinforcing the bifurcation of the aorta into the arteries leading to the lower limbs respectively, characterised by a first stent arranged inside the aorta at the level of the bifurcation and at least two cylindrical stents, each of which is positioned with a first section inside an artery branching off and with the remaining section inside the lumen of the first stent.

2. Stent as claimed in claim 1, characterised in that the first stent has a cone-shaped lumen of which the end with the largest diameter is directed away from the bifurcation.

3. Stent as characterised in claims 1 and 2, characterised in that the section of each cylindrical stent positioned inside the first stent, extends beyond the final edge with the largest diameter of this stent.

4. Stent as characterised in claims 1-3 inclusive, characterised in that the outside surface of the first stent is also cone-shaped.

5. Method for forming a Y-shaped stent inside the aorta and the arteries branching off and leading to the lower limbs respectively, comprising the following steps:
positioning a first, expandable stent by means of a positioning balloon inside the aorta above the bifurcation, and
positioning an expandable cylindrical stent in each of the arteries branching off at the bifurcation by means of a positioning balloon in such a way that this stent is positioned partially inside the artery branching off and partially extends into the lumen of the first stent.

6. Method as claimed in claim 5, characterised in that a stent with a cone-shaped lumen is employed by way of the first stent and that this stent is positioned in such a way that the end with the largest diameter is directed away from the bifurcation.

7. Method as claimed in claims 5-6 inclusive, characterised in that both cylindrical stents are positioned in such a way that the section positioned inside the lumen of the first stent extends beyond the edge with the largest diameter.
